# EUROPEAN PATENT APPLICATION

(11) **EP 4 678 208 A1**
(43) Date of publication of application: **14.01.2026**
(21) Application number: 24755916.4
(22) Date of filing: 23.01.2024
(51) Int. Cl.: A61M 15/00

(54) **POWDER AEROSOL INHALATION DEVICE**

(30) Priority: 14.02.2023 CN 202320213730 U; 14.02.2023 CN 202310108306; 14.02.2023 CN 202310108307
(71) Applicant: Hangzhou Xixi Pharmaceuticals, Hangzhou, Zhejiang 311121 (CN)
(72) Inventor: CHEN, Lan, Hangzhou, Zhejiang 310018 (CN); CHEN, Donghao, Hangzhou, Zhejiang 311121 (CN); CHEN, Hui, Hangzhou, Zhejiang 310018 (CN)
(74) Representative: Dai, Simin
(86) International application number: PCT/CN2024/073652
(87) International publication number: WO 2024/169541

(57) **Abstract**

The present disclosure discloses a dry powder inhaler, which belongs to a field of dry powder inhaler, including an upper structure, a lower structure and a piercing base with one or more piercing needles. The upper structure undergoes a longitudinal linear movement relative to the lower structure, the upper structure is formed with a capsule chamber and a piercing channel arranged laterally outside of the capsule chamber, the piercing needles undergo a lateral piercing movement along the piercing channel, a drive structure is provided between the upper structure, the lower structure, and the piercing structure, the drive structure moves with a longitudinal linear relative movement of the upper structure and the lower structure, and drives the piercing needles to pierce the capsule in the capsule chamber along the piercing channel. The driving structure includes an inclined track scheme and a linkage scheme on the lower structure, and each scheme of setting can effectively realize that the piercing needles pierce the capsule from the side along the piercing channel by pressing the lower structure and the upper structure, and the whole inhaler is beautiful and easy to operate.

## Description

### TECHNICAL FIELD

The present disclosure relates to the field of medical devices, especially relating to the field of inhalation devices for oral inhalation of dry powder to the lungs, and in particular, to a dry powder inhaler.

### BACKGROUND

Dry powder inhaler (DPI), as a new type of inhaler developed after the metered-dose inhaler (MDI) without propellants, which utilizes the patient's inspiratory airflow to drive the drug powder into the airway, with 10% to 30% of the drug deposited in the lower respiratory tract, which is slightly higher than that of the MDI. It is typically indicated to all patients over 4 years old who can cooperate with the inspiratory, with a broad demographic applicability.

Currently, there are three types of dry powder inhalers: blister-type and capsule-based and reservoir inhalers. Capsule-based inhalers can be divided into pen-shaped and elliptical grip-type inhalers, both of them have a piercing structure, especially for the elliptical grip-type inhalers. The piercing structure of the existing capsule-based dry powder inhaler is typically positioned on the bottom side, or on one side or both sides of the bottom. Take the inhaler CN215083633U and the piercing structure of the powder inhaler CN105920709B as examples, regardless of whether the configuration is single-sided or double-sided, the piercing structure comprises a lateral elastic actuation mechanism connected to piercing needles. The piercing needles are driven by the elastic actuation mechanism to pierce the capsule from its lateral aspect, with the actuation direction of the elastic actuation mechanism aligned with the movement direction of the piercing needles.

However, there are some technical problems in the capsule-based dry powder inhaler with elastic actuation mechanism and piercing structure. 1) The setting of the lateral-pressed elastic actuation mechanism requires the coordinated lateral actuation action between the index finger and thumb, which is not conducive to effective force application, presenting significant operational challenges for patients with reduced hand strength in utilizing the inhaler. 2) In addition, the setting of lateral elastic actuation mechanism of the inhaler results in a large bilateral width, affecting the overall aesthetics. The large horizontal distance is also one of the factors that increase operational complexity.

Therefore, the present disclosure is desired to provide a dry powder inhaler to solve the problems such as the technical deficiencies associated with the alignment of actuation direction of the elastic actuation mechanism and the movement direction of the piercing needles, particularly the requirement for excessive operating force and the wide horizontal width.

### SUMMARY

In order to overcome the problems raised in the prior art, the present disclosure proposes a convenient vertical pressing method that applies vertical force to solve the problem of piercing the capsule from the side.

Embodiments of the present disclosure provides a dry powder inhaler. The dry powder inhaler comprises: a piercing base, the piercing base being provided with one or more piercing needles for piercing a capsule from a side; an upper structure, the upper structure being formed with a capsule chamber and a piercing channel, wherein the capsule chamber is configured for accommodating the capsule; the piercing channel is arranged laterally outside of the capsule chamber and connected to the capsule chamber, and the piercing needles undergo a lateral piercing movement along the piercing channel; a lower structure for accommodating a portion of the upper structure, the upper structure being subjected to a longitudinal linear movement relative to the lower structure; and a drive structure arranged on at least one of the upper structure and the lower structure, the piercing base being driven by the drive structure to undergo movement. The drive structure moves with the upper structure and the lower structure undergoing a longitudinal linear relative approach movement, the drive structure drives the piercing base to move in a direction of the piercing channel, the piercing needles move with the piercing base along the piercing channel to pierce the capsule in the capsule chamber; the drive structure moves with the upper structure and the lower structure undergoing a longitudinal linear relative separation movement, the drive structure drives the piercing base to move in the direction of the piercing channel, and the piercing needles leave the capsule chamber with the piercing base along the piercing channel.

Preferably, the direction of the piercing channel is horizontal or inclined upward or downward.

The drive structure includes an inclined track arranged within the lower structure for a tilting movement of the piercing base, the inclined track being tilted in a direction gradually inclined from a position at an upper edge of the lower structure to a lower portion of the lower structure and toward the capsule chamber, when the upper structure and the lower structure are subjected to the longitudinal linear relative approach movement, the piercing base is driven by the inclined track and guided by the piercing channel to move toward the capsule chamber, and the piercing needles enter the capsule chamber along the piercing channel to complete piercing of the capsule.

Preferably, the piercing channel is a straight channel and the piercing needles are straight needles; and the piercing needles are contained in a portion of the piercing channel at any time.

Preferably, the direction of the piercing channel is horizontal or inclined upward or downward, and a direction of the piercing movement along the piercing channel is combined with a direction of the longitudinal linear movement between the upper structure and lower structure to form a combined movement direction along a direction of the inclined track.

Preferably, a combined movement distance of the piercing distance of the piercing base in the piercing channel and the longitudinal linear movement distance is a tilting movement distance on the inclined track.

Preferably, after an incline angle of the inclined track is determined, a proportional relationship between the piercing distance and the longitudinal linear movement distance between the upper structure and the lower structure is determined based on the incline angle of the inclined track. Alternatively, the incline angle is determined based on the desired longitudinal linear movement distance and the piercing distance of the piercing channel.

Preferably, the direction of the piercing channel is horizontal, a length of the inclined track is L1, a piercing distance of the piercing needles is L2, and a longitudinal linear movement distance between the upper structure and the lower structure is L3, and a relationship between the length L1, the piercing distance L2, and the longitudinal linear movement distance L3 is represented as that the length L1 is greater than a length of a hypotenuse of a right triangle formed by L2 and L3.

Preferably, the inclined track is groove-type inclined tracks provided on front and rear sidewalls of the lower structure, the piercing base is provided with protruding columns extending into the groove-type inclined tracks.

Preferably, a groove-type opening of the groove-type inclined track is a square or "T" shaped opening.

Preferably, the inhaler is of single-side piercing type, one piercing base is provided, two groove-type inclined tracks are provided on the front and rear sidewalls of the lower structure, the protruding columns extending into the two groove-type inclined tracks are provided on front and rear sides of the piercing base, the piercing base is provided with two piercing needles arranged in parallel, and two piercing channels arranged in parallel are provided on the same side in correspondence.

Optionally, the inhaler is of double-side piercing type, two piercing bases are provided, four groove-type inclined tracks are provided correspondingly on the front and rear sidewalls of the lower structure, and the protruding columns extending into the two of the four groove-type inclined tracks are provided on front and rear sides of each piercing base; preferably, the two piercing bases and the four groove-type inclined tracks are symmetrically provided around the capsule chamber; preferably, one piercing needle is provided on one piercing base, and two piercing channels arranged in parallel or in the same straight line are provided on both sides of the inhaler.

The present disclosure also provides a slope-type embodiment of the inclined track, the inclined track is a slope-type inclined track provided on left and right sidewalls of the lower structure, a movement track of the slope-type inclined track is an inclined surface of the slope-type inclined track, and the piercing base is provided with an anastomosing surface that fits into the inclined surface of the slope-type inclined track; the anastomosing surface is provided on a main base, and a needle holder with the piercing needles is provided above the main base;

When the inhaler is of single-side piercing type, the inhaler includes a slope-type inclined track and the piercing base, wherein two piercing needles arranged in parallel are provided on the piercing base, and two piercing channels arranged in parallel are provided on the same side of the dry powder inhaler; or when the inhaler is of double-side piercing type, two slope-type inclined tracks are symmetrically provided on the left and right sidewalls of the lower structure and two piercing bases corresponding to the two slope-type inclined tracks are provided, one piercing needle is provided on the piercing base, and two piercing channels arranged in parallel or in the same straight line are provided on both sides of the dry powder inhaler.

Preferably, the inhaler further includes a fitting structure for fitting a main base on the inclined track, the fitting structure includes a spring structure providing elasticity on side, one end of the spring structure being attached to a lower end of the upper structure and the other end of the spring structure being attached to a side of the main base; or the fitting structure is a T-shaped limiting groove arranged on the inclined surface, and the fitting structure further including an adapted T-shaped protrusion extending from the anastomosing surface, so that the anastomosis surface and the inclined surface are always fitted.

The present disclosure also provides a linkage structure embodiment, the drive structure includes a linkage structure connected to the piercing base and a hinge structure; and the hinge structure is configured to articulate the linkage structure with the lower structure and the piercing base.

The linkage structure includes at least one main connecting rod obliquely connected to the piercing base. One end of the main connecting rod is hinged to a sidewall of the lower structure through the hinge structure, and the other end of the main connecting rod is hinged to the piercing base through the hinge structure; the main connecting rod is tilted in a direction gradually tilting upwardly from a position at the upper edge of the lower structure and toward the capsule chamber.

When the upper structure and the lower structure undergo the longitudinal linear relative movement, the main connecting rod rotates with the longitudinal linear relative movement, the main connecting rod rotates to drive the piercing base to move in the direction of the piercing channel, and the piercing needles enter the capsule chamber along the piercing channel with the piercing base to pierce the capsule in the capsule chamber.

Preferably, the direction of the piercing channel is horizontal or inclined upward or downward, taking the direction of the piercing channel being horizontal as an example, an incline angle of the main connecting rod is a, a length of the main connecting rod is L1, a piercing distance of the piercing needles is L2, and a relationship between the incline angle a of the main connecting rod, the length L1 of the main connecting rod, the piercing distance L2, and the longitudinal linear movement distance L3 of the upper structure and the lower structure is represented as L1≥L2+L3*cota.

Preferably, an anti-separation structure is provided to solve the anti-separation problem after a combination of the upper structure and the lower structure.

Preferably, on top of the upper structure, a structure is set to facilitate the realization of push operation to ensure the convenience of movement operation.

Preferably, mutually socketed lumen structures are provided at the bottom of the upper structure and at a bottom surface of the lower structure in correspondence, the lumen structures consist of an upper lumen at the bottom of the upper structure and a lower lumen extending from the bottom surface of the lower structure. The upper lumen is socketed on an outer side of the lower lumen or the upper lumen extends into an inner side of the lower lumen, and the reset spring is provided in a center of the upper lumen and the lower lumen. Alternatively, a bottom cavity for movement of a bottom structure of the upper structure is provided within the lower structure, the bottom structure is adapted to the bottom cavity in shape, the reset spring is provided in the bottom cavity, and a portion of the bottom structure is provided within the bottom cavity. Setting up in this way can also effectively achieve guiding the occurrence of the longitudinal linear movement.

Beneficial effects of the present disclosure include: for the arrangement of the drive structure, the arrangement of the piercing base and the inclined track or the arrangement of the linkage structure realizes that the piercing needles pierce the capsule from the side through the combination with the longitudinal linear movement of the upper structure and the lower structure. The design concept of the inhaler provided in the embodiments of the present disclosure is a new concept for accomplishing the capsule piercing action of the dry powder inhaler by a new form of movement. By such an arrangement, the single-handed horizontal force mode is converted to the single-handed longitudinal force mode, and the longitudinal vertical force mode is easier to operate than the horizontal force mode. Moreover, the aesthetics of the inhaler is higher because the structures are set up in the upper structure and the lower structure. In addition, the overall transverse width of the inhaler can be reduced by converting the direction of the force and it is more convenient to hold the inhaler.

The arrangement of the groove-type inclined tracks on the front and rear sidewalls of the lower structure and symmetrically arrangement of the inclined tracks around the capsule chamber can symmetrically pierce the capsule from both sides, which is safe and effective during dry powder inhalation.

The arrangement of the slope-type inclined track on the left and right sidewalls of the lower structure is also a good technical solution that can realize the corresponding function similar to that of the groove-type inclined track, with remarkable technical effect.

The arrangement of the linkage structure connected to the upper structure in the lower structure can also well realize the effect of piercing the capsule in the capsule chamber along the piercing channel under the premise of guaranteeing that the main connecting rod moves in a motion plane and the idea of arranging specific structures.

### BRIEF DESCRIPTION OF THE DRAWINGS

The present disclosure is further described in terms of exemplary embodiments. These exemplary embodiments are described in detail with reference to the drawings. These embodiments are non-limiting embodiments, in which like reference numerals represent similar structures throughout the drawings, wherein:
Fig. 1 is a schematic diagram illustrating an overall structure of a dry powder inhaler without a protective cap according to some embodiments of the present disclosure;
Fig. 2 is a schematic diagram illustrating an overall longitudinal cross-sectional structure of the dry powder inhaler without a protective cap according to some embodiments of the present disclosure;
Fig. 3 is a schematic diagram illustrating a structure of an inhalation channel section according to some embodiments of the present disclosure;
Fig. 4 is a schematic diagram illustrating a structure of a nozzle section according to some embodiments of the present disclosure;
Fig. 5 is a schematic diagram illustrating a left-right longitudinal cross-sectional structure of a lower structure according to some embodiments of the present disclosure;
Fig. 6 is a schematic diagram illustrating a front-to-rear longitudinal cross-sectional structure of an overall structure of an anti-separation structure with a limit rod according to some embodiments of the present disclosure;
Fig. 7 is a schematic diagram illustrating a portion of an upper structure and the lower structure according to some embodiments of the present disclosure;
Fig. 8 is a schematic diagram illustrating a partial longitudinal cross-sectional structure of the upper structure and the lower structure of the groove-type inclined track embodiment according to some embodiments of the present disclosure;
Fig. 9 is a schematic diagram illustrating the upper structure and a piercing base of the groove-type inclined track embodiment according to some embodiments of the present disclosure;

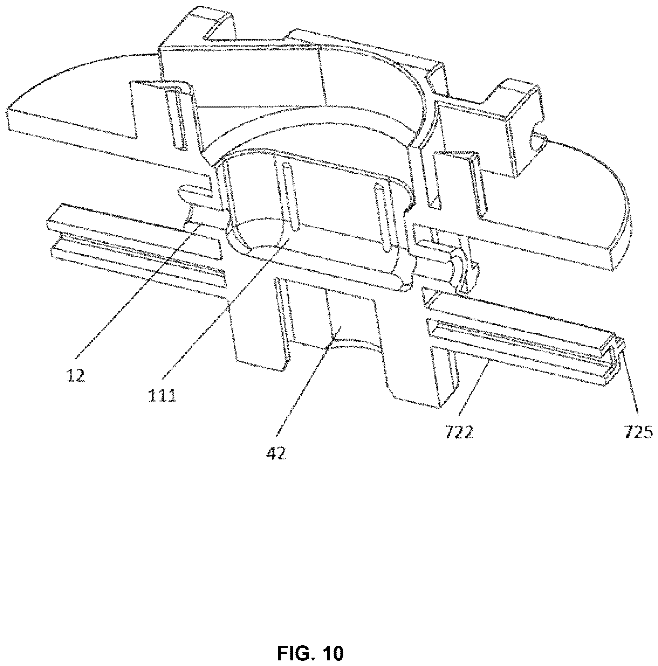

Fig. 10 is a schematic diagram illustrating the upper structure of the groove-type inclined track embodiment according to some embodiments of the present disclosure;
Fig. 11 is a schematic diagram illustrating a structure of the piercing base of the groove-type inclined track embodiment according to some embodiments of the present disclosure;
Fig. 12 is a schematic diagram illustrating a structure of a groove-type inclined track embodiment in an unpierced state according to some embodiments of the present disclosure;
Fig. 13 is a schematic diagram illustrating a structure of a groove-type inclined track embodiment in a pierced state according to some embodiments of the present disclosure;
Fig. 14 is a schematic structural diagram illustrating an upper structure and a lower structure of a slope-type inclined track embodiment, with the upper structure not showing a finger placement structure according to some embodiments of the present disclosure;
Fig. 15 is a schematic diagram illustrating a left-right longitudinal cross-sectional structure of an upper structure and a lower structure of the slope-type inclined track embodiment, with the upper structure not showing the finger placement area according to some embodiments of the present disclosure;
Fig. 16 is a schematic diagram illustrating a left-right longitudinal cross-sectional structure of the lower structure of the slope-type inclined track embodiment according to some embodiments of the present disclosure;
Fig. 17 is a schematic diagram illustrating a structure of a piercing base with parallel rods of the slope-type inclined track embodiment according to some embodiments of the present disclosure;
Fig. 18 is a schematic diagram illustrating a partially enlarged structure of a T-shaped limiting groove of the slope-type inclined track embodiment according to some embodiments of the present disclosure;
Fig. 19 is a schematic diagram illustrating a piercing base with T-shaped protrusions of the slope-type inclined track embodiment according to some embodiments of the present disclosure;
Fig. 20 is a schematic diagram illustrating a left-right longitudinal cross-sectional structure of the slope-type inclined track embodiment in an unpierced state according to some embodiments of the present disclosure;
Fig. 21 is a schematic diagram illustrating a left-right longitudinal cross-sectional structure of the slope-type inclined track embodiment in a pierced state according to some embodiments of the present disclosure;
Fig. 22 is a schematic diagram illustrating a structure of an upper structure and a lower structure of a tilting main connecting rod embodiment, with the upper structure not showing a finger placement area according to some embodiments of the present disclosure;
Fig. 23 is a schematic diagram illustrating a left-right longitudinal cross-sectional structure of an upper structure and a lower structure of the tilting main connecting rod embodiment, with the upper structure not showing the finger placement area according to some embodiments of the present disclosure;
Fig. 24 is a schematic diagram illustrating a structure of the upper structure, a linkage structure, and piercing needles in a combined state of the tilting main connecting rod embodiment according to some embodiments of the present disclosure;
Fig. 25 is a schematic diagram illustrating a structure of the linkage structure and the piercing needles in a combined state of the tilting main connecting rod embodiment according to some embodiments of the present disclosure;
Fig. 26 is a schematic diagram illustrating a left-right longitudinal cross-sectional structure of the tilting main connecting rod embodiment in an unpierced state according to some embodiments of the present disclosure;
Fig. 27 is a schematic diagram illustrating a left-right longitudinal cross-sectional structure of the tilting main connecting rod embodiment in a pierced state according to some embodiments of the present disclosure; and
Fig. 28 is a schematic diagram illustrating a relationship between an incline angle of the main connecting rod, a length of the main connecting rod, a piercing distance of the piercing needles, and a longitudinal linear movement distance between the upper structure and the lower structure according to some embodiments of the present disclosure.

In the figures, 1, upper structure; 111, capsule chamber; 112, rotary chamber; 1121, airflow channel opening; 12, piercing channel; 2, lower structure; 21, accommodating chamber; 3, piercing needle; 31, needle holder; 4, reset spring; 41, upper lumen; 42, lower lumen; 51, limit rod; 52, locking protrusion; 53, movement groove; 54, limit protrusion; 55, guide groove; 56, guide surface; 571, direction-guiding groove; 572, direction-guiding column; 61, operation table; 611, anti-slip pattern; 612, anastomosis table; 62, inhalation channel section; 621, columnar channel tube; 63, nozzle section; 631, anastomosis tube; 64, protective cap; 71, groove-type inclined track; 711, guide opening; 72, piercing base; 721, protruding column; 722, cylindrical body; 723, columnar cavity; 724, secondary adaptation groove; 725, secondary adaptation protrusion; 81, slope-type inclined track; 811, inclined surface; 812, anastomosing surface; 82, main base; 831, spring structure; 832, parallel lumen; 833, parallel rod; 841, T-shaped limiting groove; 842, T-shaped protrusion; 851, bottom structure; 852, bottom cavity; 853, secondary adaptation cavity; 854, secondary adaptation rod; 91, main connecting rod; 921, running channel; 922, plate structure; 93, maintaining connecting rod; 94, connecting portion of connecting rod.

### DETAILED DESCRIPTION

The technical solutions in the embodiments of the present disclosure are hereinafter described clearly and completely by means of particular specific embodiments, and it is obvious that the described embodiments are only a part of the embodiments of the present disclosure and not all of them, and a person skilled in the art can easily understand other advantages and efficacies of the present disclosure by the contents disclosed in the present disclosure. The present disclosure may also be implemented or applied in different other specific embodiments, and the following embodiments and features in the embodiments may be combined with each other in a way that does not conflict with each other, and based on the embodiments of the present disclosure, those skilled in the art may, without making any creative efforts, realize the other embodiments belonging to the scope of protection of the present disclosure.

### Embodiment 1

A dry powder inhaler includes an upper structure 1, a lower structure 2, and a piercing base 72. The upper structure 1is subjected to a longitudinal linear movement relative to the lower structure 2. The piercing base 72 includes one or more piercing needles 3. The upper structure 1 is formed with a capsule chamber 111 and a piercing channel 12 on the outside of the capsule chamber 111. The piercing channel 12 provides a piercing trajectory for the piercing needles 3 to undergo a lateral piercing movement. The upper structure 1 undergoes a longitudinal linear relative movement with the lower structure 2, which simultaneously drives the piercing base 72 to pierce the capsule in the capsule chamber 111 through the piercing channel 12. The piercing channel 12 is oriented in a horizontal direction or an inclined upward direction or an inclined downward direction to enable piercing of the capsule to be accomplished from a horizontal side, an upper side, or a lower side. The inhaler is further provided with a drive structure, which is provided on the upper structure 1 and/or the lower structure 2; and the piercing base 72 is driven by the drive structure to undergo a movement.

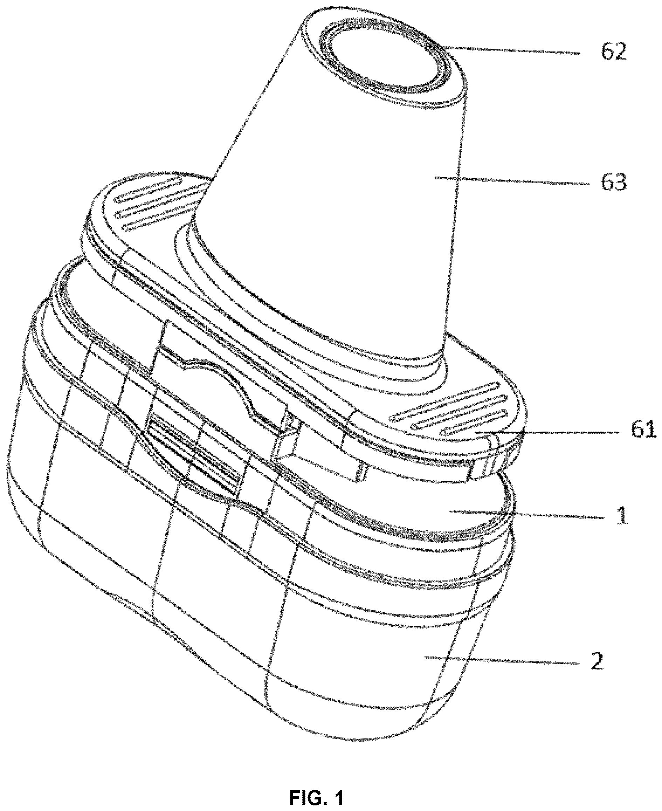

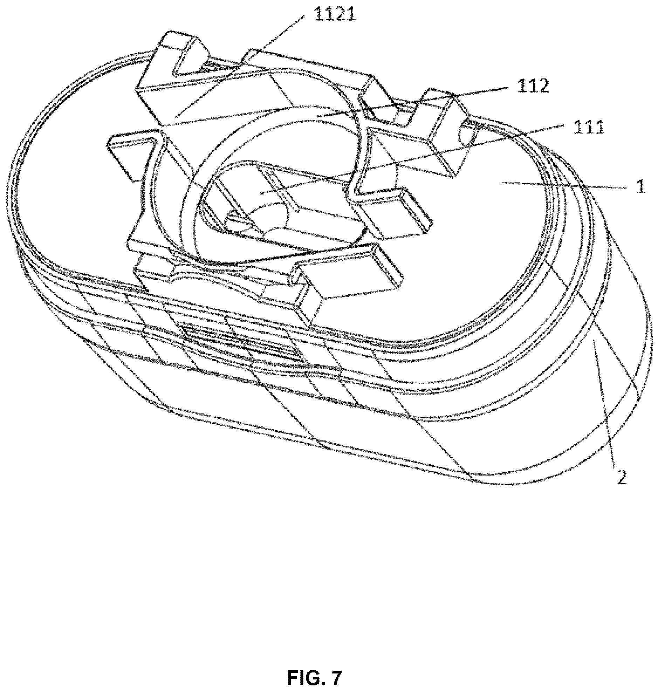

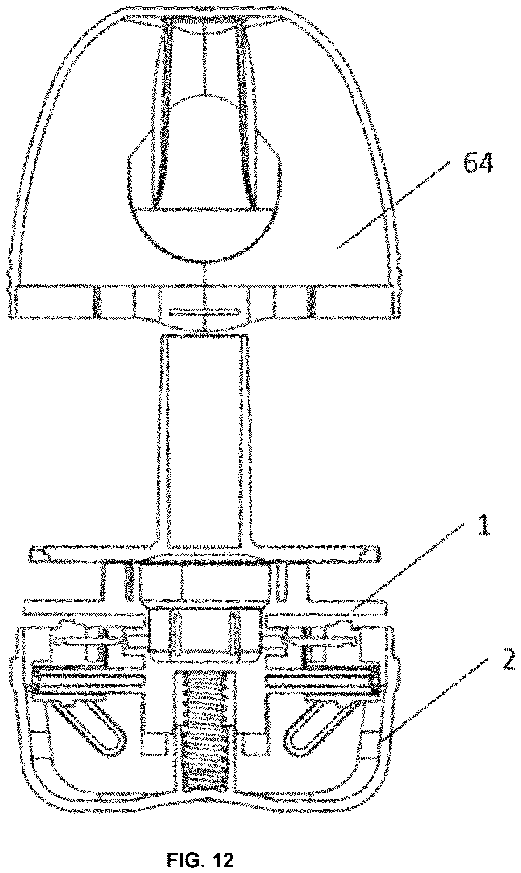

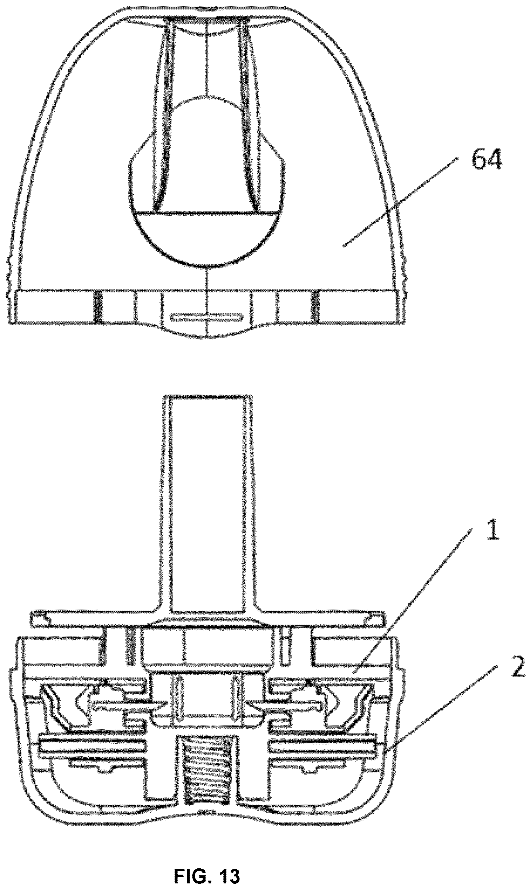

The drive structure moves with the longitudinal linear relative approach movement of the upper structure 1 and the lower structure 2, while the drive structure drives the piercing base 72 to move in the direction of the piercing channel 12, and the piercing needles 3 move along the piercing channel 12 with the piercing base 72 into the capsule chamber 111 to pierce the capsule in the capsule chamber 111. The drive structure moves with the longitudinal linear relative separation movement of the upper structure 1 and the lower structure 2, and the drive structure drives the piercing base 72 to move in the direction of the piercing channel 12, and the piercing needles 3 leave the capsule chamber 111 along the piercing channel 12 with the piercing base 72, as shown in Figs.1, 6, 7, 12, and 13.

Shapes and a movement relationship of the lower structure 2 and the upper structure 1 may be as follows: the lower structure 2 is formed with an accommodating chamber 21, the accommodating chamber 21 is configured to hold the upper structure 1 and the piercing base 72, and the upper structure 1 undergoes the longitudinal linear relative movement within the accommodating chamber 21 of the lower structure 2. The upper structure 1 further includes a rotary chamber 112 disposed above the capsule chamber 111, and inclined airflow channel openings 1121 are disposed on both sides of the rotary chamber 112. A reset structure is provided for realizing a motion reset of the upper structure 1 and the lower structure 2. Preferably, the reset structure is a reset spring 4, as shown in Figs. 1 and 6-8.

### Embodiment 2

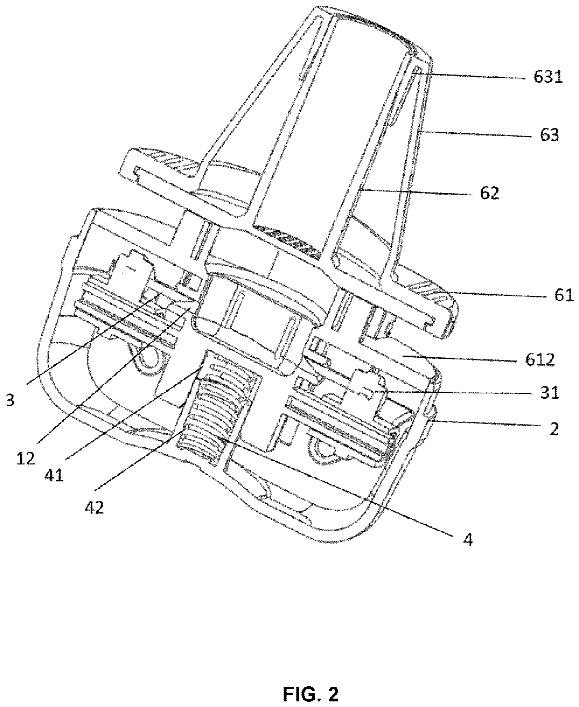

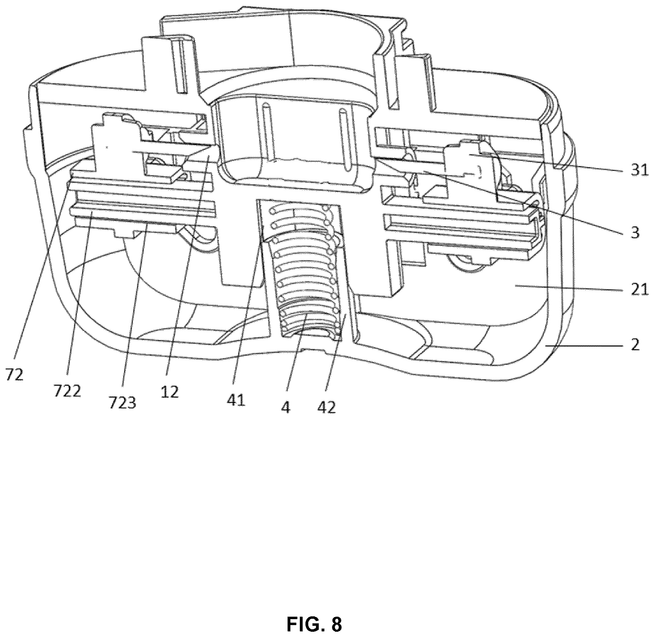

On the basis of Embodiment 1, the piercing channel 12 is a straight channel and the piercing needles 3 are straight needles; the piercing needles 3 are contained in a portion of the piercing channel 12 at any time when the upper structure 1 undergoes the longitudinal linear movement with the lower structure 2. The drive structure includes an inclined track provided within the lower structure 2 for realizing a tilting movement of the piercing base 72, and the inclined track is tilted in a direction gradually inclined from a position at the upper edge of the lower structure 2 to the lower portion of the lower structure 2 and towards the capsule chamber 111, which can realize that when the lower structure 2 undergoes an upward longitudinal linear relative approach movement, the piercing base 72 moves towards the capsule chamber 111 to complete the piercing of the capsule. See Figs. 2 and 8.

The piercing channel 12 is oriented in a horizontal direction or an inclined upward direction or an inclined downward direction. A direction of the piercing movement of the piercing channel 12 and a direction of the longitudinal linear movement of the upper structure 1 and the lower structure 2 may be combined into a direction of combined movement along the inclined track. This arrangement can effectively ensure that various settings of the inclined track are possible. See Fig. 2.

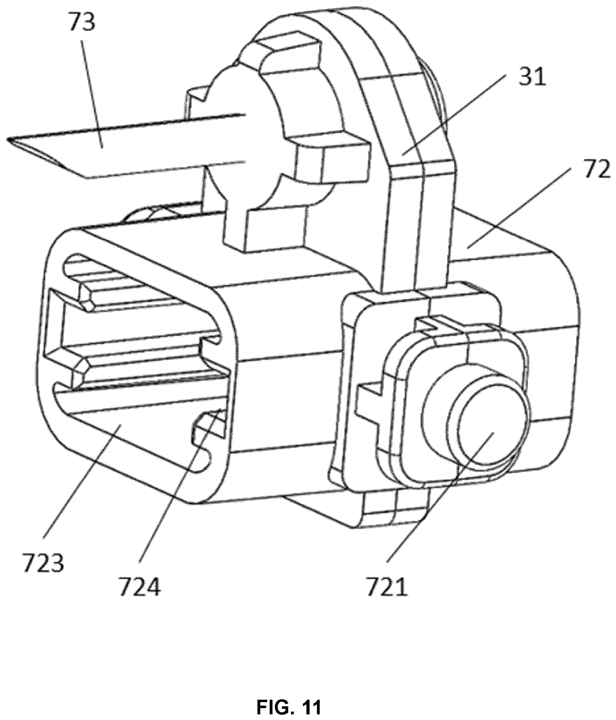

The combined movement distance of the piercing distance of the piercing base 72 in the piercing channel 12 and the longitudinal linear movement distance is a tilting movement distance on the inclined track. After an incline angle of the inclined track is determined, a proportional relationship between the piercing distance and the longitudinal linear movement distance of the upper structure 1 and the lower structure 2 is determined based on the incline angle of the inclined track. Alternatively, the incline angle is calculated based on the desired longitudinal linear movement distance and the piercing distance of the piercing channel 12. This approach enables a more rapid determination of various parameters of the various structures, see Figs. 8 and 11.

Taking the direction of the piercing channel 12 being horizontal as an example, a length of the inclined track is L1, a piercing distance of the piercing needles is L2, and a longitudinal linear movement distance between the upper structure and the lower structure is L3, and a relationship between the length L1, the piercing distance L2, and the longitudinal linear movement distance L3 is represented as that the length L1 is greater than a length of a hypotenuse of a right triangle formed by L2 and L3. See Fig. 2.

### Embodiment 3

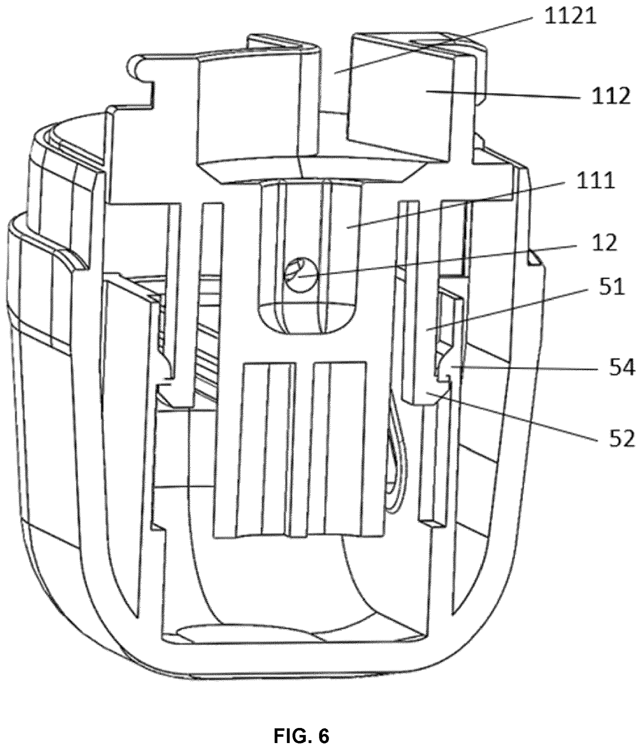

On the basis of Embodiment 2, the first embodiment of the inclined track is as follows: the inclined track is a groove-type inclined track 71 provided on the front and rear sidewalls of the lower structure 2, and a protruding column 721 extending into the groove-type inclined track 71 is provided on the piercing base 72, a groove-type opening of the groove-type inclined track 71 is a square or "T" shaped opening, and one or two piercing bases 72 are provided around the capsule chamber 111, as shown in Figs. 6-9.

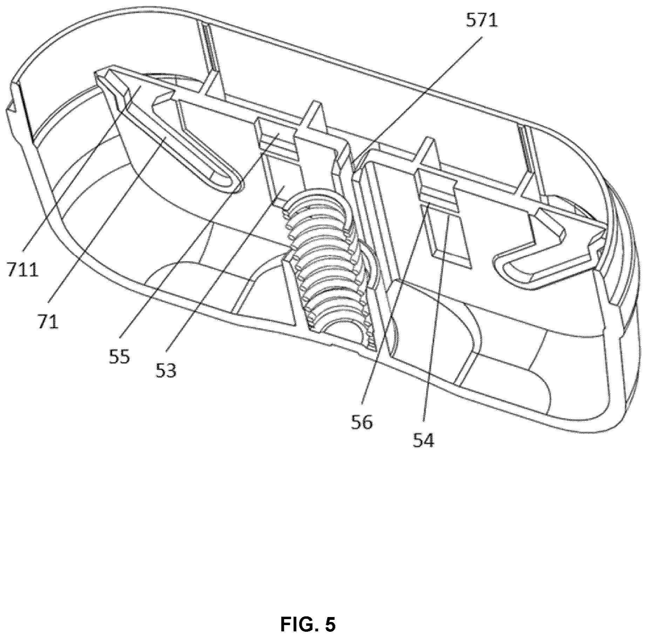

When the inhaler is a single-side piercing type, one piercing base 72 is provided, the front and rear sidewalls of the lower structure 2 are provided with two groove-type inclined tracks 71, the front and rear sides of the piercing base 72 are provided with protruding columns 721 that extend into the groove-type inclined tracks 71, and the piercing base 72 is provided with two parallel piercing needles 3, and two parallel piercing channels 12 are correspondingly provided on the same side of the inhaler, referring to Figs. 5, 8, and 9.

When the inhaler is a double-side piercing type, two piercing bases 72 are provided, and four groove-type inclined tracks 71 are provided, the front and rear sides of each of the piercing bases 72 are provided with protruding columns 721 that extend into the groove-type inclined tracks 71. Preferably, the two piercing bases 72 and the four groove-type inclined tracks 71 are symmetrically provided around the capsule chamber 111. One piercing needle 3 is provided on one piercing base 72, and two piercing channels 12 arranged in parallel or in the same straight line are provided on both sides of the inhaler, referring to Figs. 5-9.

The inhaler is further provided with a motion-guiding structure for ensuring movement of the piercing base 72 along the piercing channel 12, the piercing base 72 is provided with an adapting portion in combination with the motion-guiding structure, and the adapting portion moves on the motion-guiding structure. The motion-guiding structure is a cylindrical body 722 or a columnar cavity 723 protruding from the left and right sidewalls of the upper structure 1 or the lower structure 2, and the motion-guiding structure is oriented in a direction that is parallel to the piercing channel 12. Preferably, the motion-guiding structure is the cylindrical body 722 or the columnar cavity 723 extending from the sidewall of the upper structure 1, referring to Figs. 2 and 8.

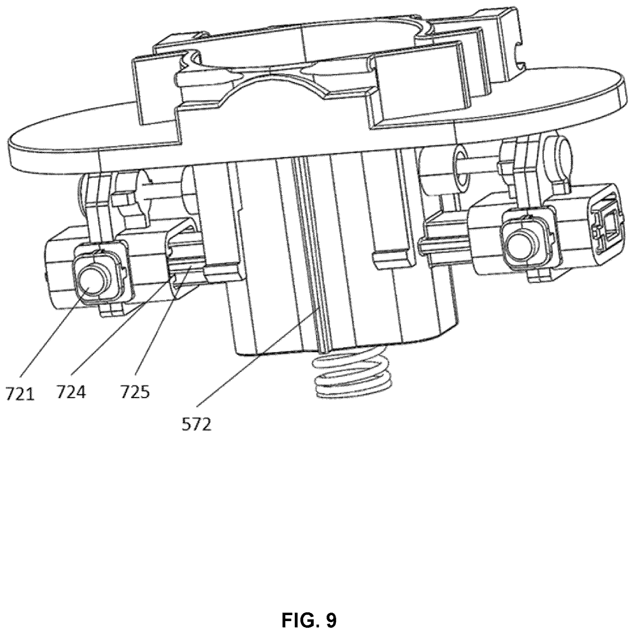

The motion-guiding structure is adapted to the adapting portion in such a manner as follows: when the motion-guiding structure is a cylindrical body 722, the adapting portion is the columnar cavity 723 that is socketed onto the outside of the cylindrical body 722, and the protruding column 721 protrudes outward from the front and rear walls of the columnar cavity 723. In other embodiments, when the motion-guiding structure is a columnar cavity 723, the adapting portion is a cylindrical body 722 disposed inside the columnar cavity 723, the protruding column 721 extends out from the outside of the front and rear walls of the cylindrical body 722, and the transverse penetration notch is arranged on the front and rear sidewalls of the columnar cavity 723 to realize the protrusion of the protruding column 721 and ensure the protruding movement, referring to Figs. 8 and 9.

In order to better realize the adaptation of the motion-guiding structure and the adapting portion, a secondary adaptation groove 724 is provided within the columnar cavity 723 and a secondary adaptation protrusion 725 is provided on the cylindrical body 722. Preferably, the secondary adaptation groove 724 is symmetrically disposed on the inner sides of the front and rear sidewalls or on the inner sides of the top and bottom sidewalls of the columnar cavity 723. In order to better set the piercing needles 3 on the piercing base 72, the piercing base 72 is provided with a needle holder 31 accommodating the piercing needles 3. The piercing needles 3 are integrally connected to the needle holder 31 or the piercing needles 3 are combined with the needle holder 31, and the position remains unchanged after the combination. A positional relationship between the needle holder 31 and the motion-guiding structure may be set as follows: the needle holder 31 is set above the motion-guiding structure and the needle holder 31 is smaller in size than the motion-guiding structure, which ensures the stabilization of the structure of the piercing base 72. In order to facilitate the realization of combining a columnar base into the groove-type inclined track 71, a guide opening 711 is provided at the uppermost part of the lower structure 2, the guide opening 711 is larger in size than the inclined track opening, and the guide opening 711 is a triangular opening, referring to Figs. 5, 8, and 9.

### Embodiment 4

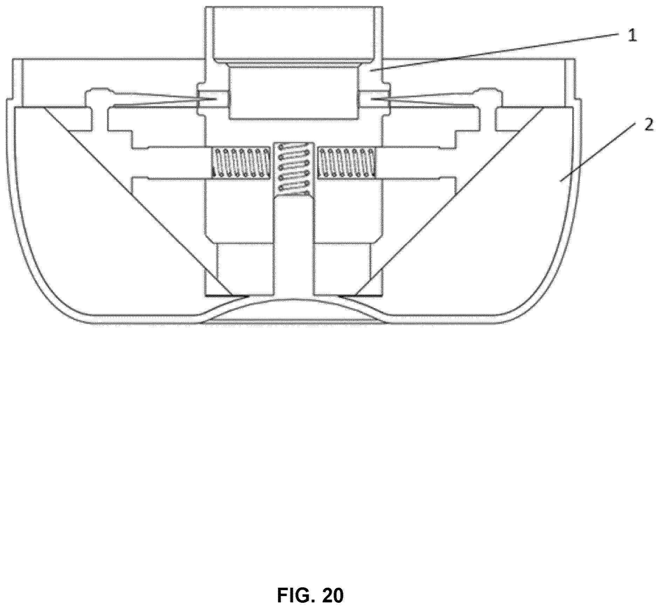

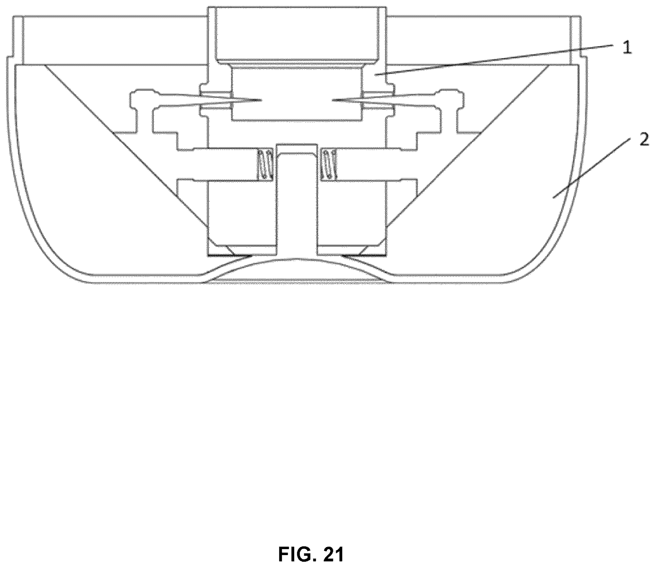

The second embodiment of the inclined track on the basis of Embodiment 2 is as follows: the inclined track may also be a slope-type inclined track 81 provided on the left and right sidewalls of the lower structure 2, and the movement track of the slope-type inclined track 81 is an inclined surface 811 of the slope-type inclined track 81, and the needle holder 31 is provided with an anastomosing surface 812 that fits with the inclined surface 811 of the slope-type inclined track 81, the piercing base 72 includes a main base 82; the anastomosing surface 812 is provided on the main base 82, and the needle holder 31 with the piercing needles 3 is provided above the main base 82, referring to Figs. 8, 14-15, and 20-21.

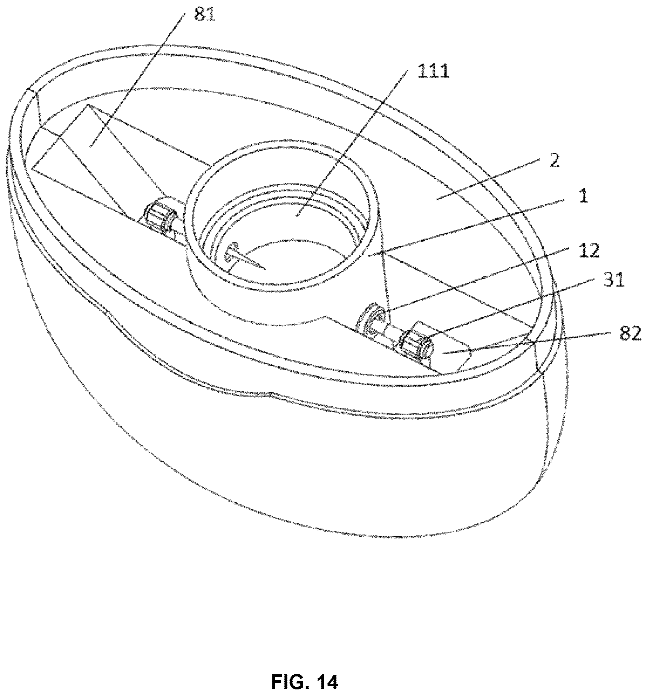

When the inhaler is a single-side piercing type, the inhaler includes one slope-type inclined track 81 and one main base 82, and two parallel piercing needles 3 are provided on one needle holder 31, and two piercing channels 12 arranged in parallel are provided on the same side of the inhaler. When the inhaler is a double-side piercing type, two slope-type inclined tracks 81 are symmetrically provided on the left and right sidewalls of the lower structure 2, and two main bases 82 corresponding to the two slope-type inclined tracks 81 are provided, one needle holder 31 is provided with one piercing needle 3, and two piercing channels 12 arranged in parallel or in the same straight line are provided on both sides of the inhaler, referring to Figs. 8 and 14.

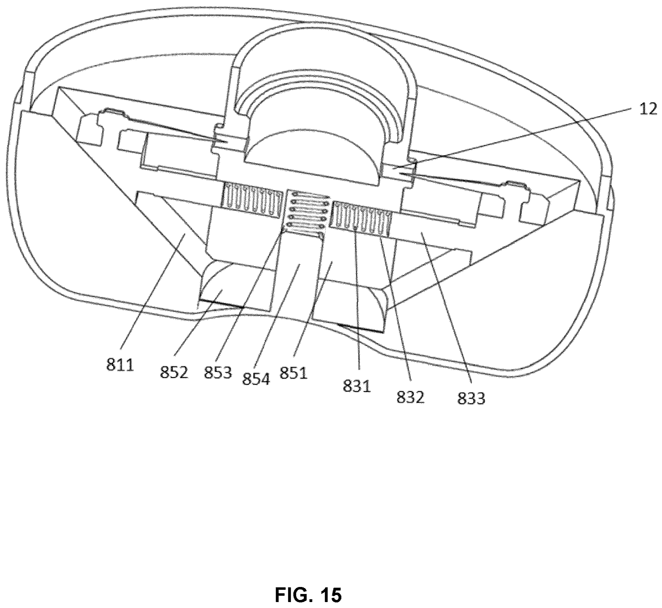

The inhaler further includes a fitting structure for fitting the main base 82 on the inclined track. The first embodiment of the fitting structure is as follows: the fitting structure includes a spring structure 831 providing elasticity on the side. One end of the spring structure 831 is attached to a lower end of the upper structure 1, and the other end of the spring structure 831 is attached to a side of the main base 82. The arrangement of the spring structure 831 can maintain the main base 82 to fit on the inclined track by the elasticity to ensure the mating movement of the parts, referring to Figs. 14 and 15.

In order to further maintain the elasticity stability of the spring structure 831, the spring structure 831 is confined within a parallel lumen 832 that is parallel to the piercing channel 12. The parallel lumen 832 includes the parallel lumen 832 attached to the lower portion of the upper structure 1 and a parallel rod 833 attached to a side portion of the main base 82, and the parallel rod 833 always has a socketed section with the parallel lumen 832. This arrangement ensures that the spring structure 831 provides a stabilizing force. The parallel lumen 832 may protrude in the lower portion of the upper structure 1 or be set on the sidewall of the upper structure 1, referring to Figs. 14 and 15.

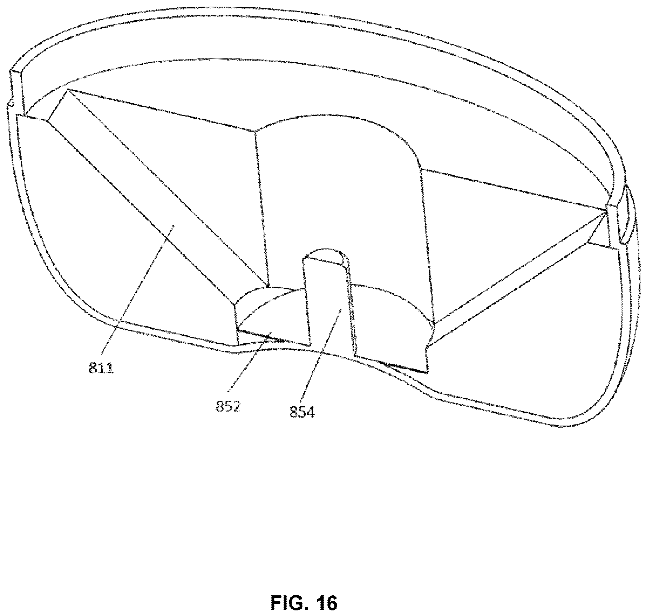

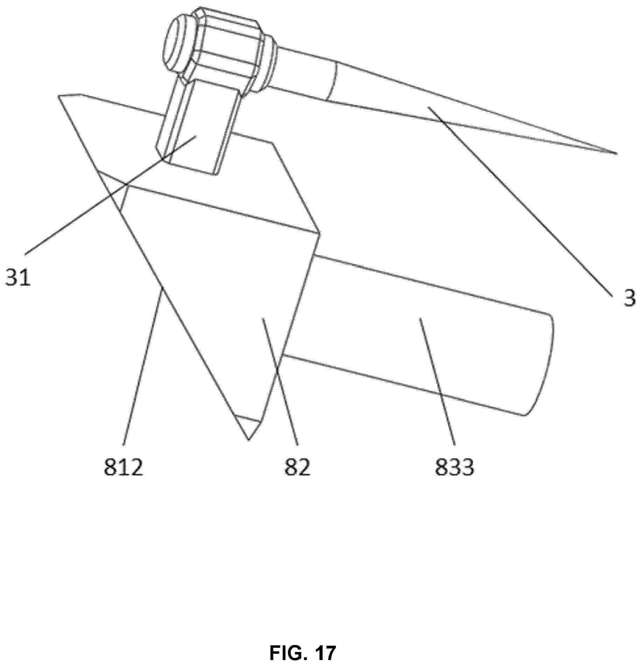

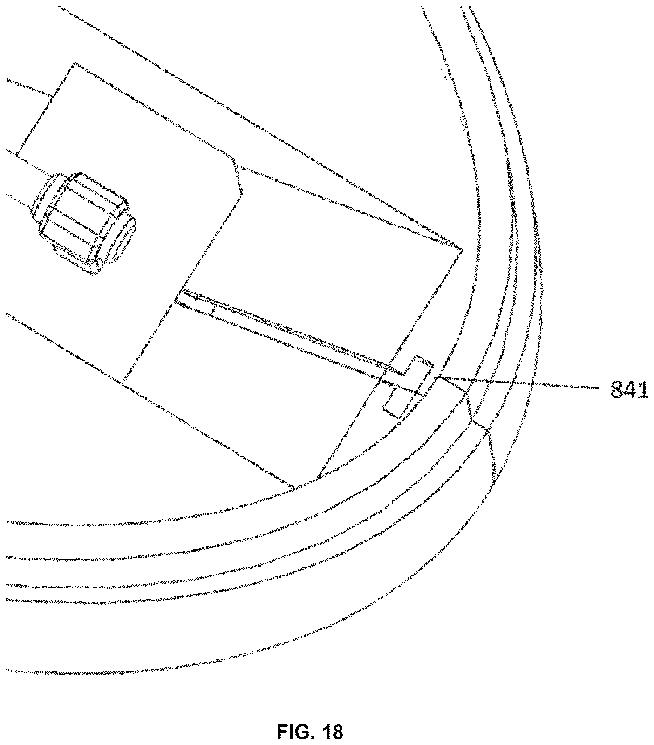

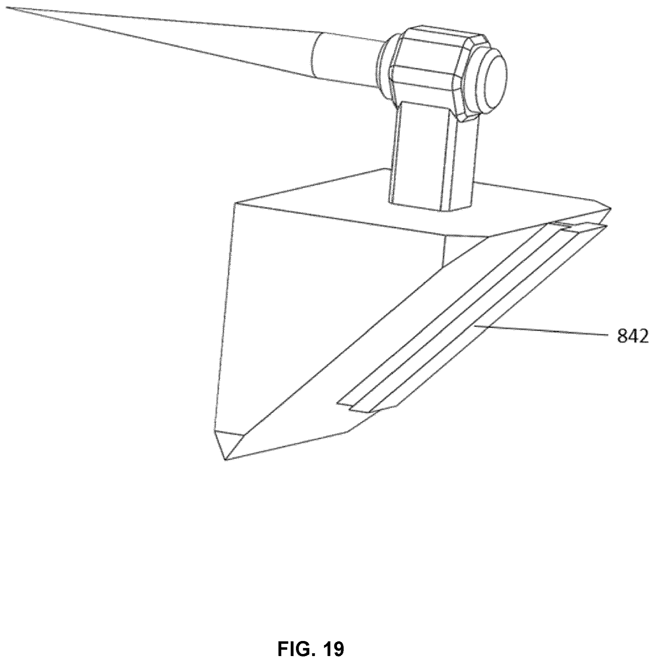

The second embodiment of the fitting structure is as follows: the fitting structure is a T-shaped limiting groove 841 provided on the inclined surface 811, and the fitting structure further includes a T-shaped protrusion 842 protruding out of the anastomosing surface 812, the T-shaped protrusion 842 being adapted to the T-shaped limiting groove 841, which can achieve that the anastomosing surface 812 is always fitted with the inclined surface 811. In this embodiment, the parallel lumen 832 may also be provided to increase structural stability, the parallel lumen 832 includes the parallel lumen 832 attached to the lower portion of the upper structure 1 and a parallel rod 833 attached to the side of the main base 82, and the parallel lumen 832 and the parallel rod 833 have a socketed section. Such arrangement ensures that the spring structure 831 provides a stable force. The parallel lumen 832 may protrude in the lower portion of the upper structure 1 or be set on the sidewall of the upper structure 1, referring to Figs. 14-19.

There may also be an embodiment for improving structural stability, a secondary adapter structure may be provided within the parallel lumen 832 and the parallel rod 833, which includes a secondary adaptation groove 724 and a secondary adaptation protrusion 725 correspondingly. The secondary adapter structure can enhance the adaptation between the parallel lumen 832 and the parallel rod 833, increasing the structural stability, referring to Figs. 9 and 15.

### Embodiment 5

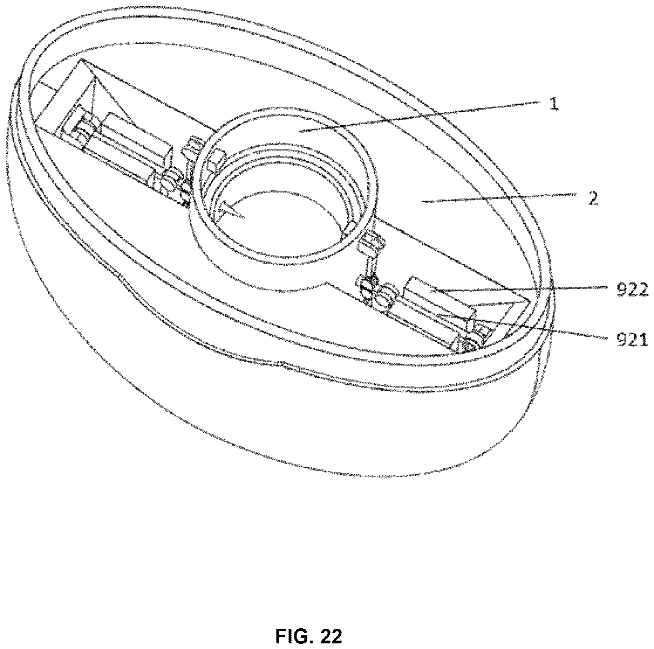

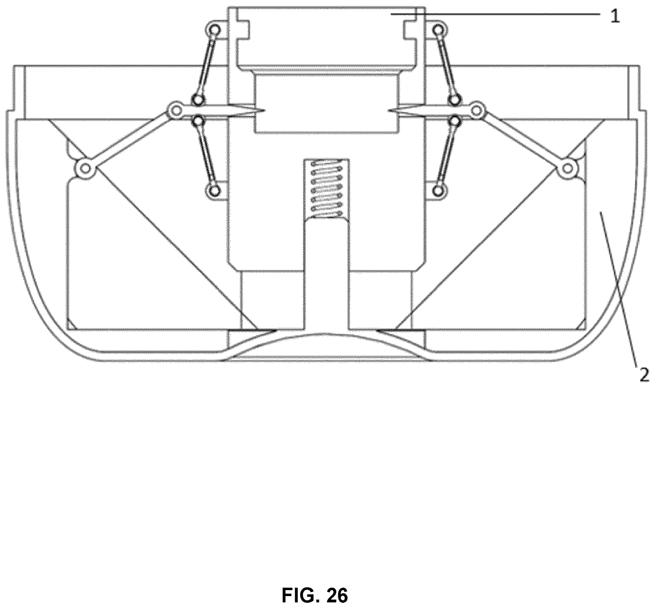

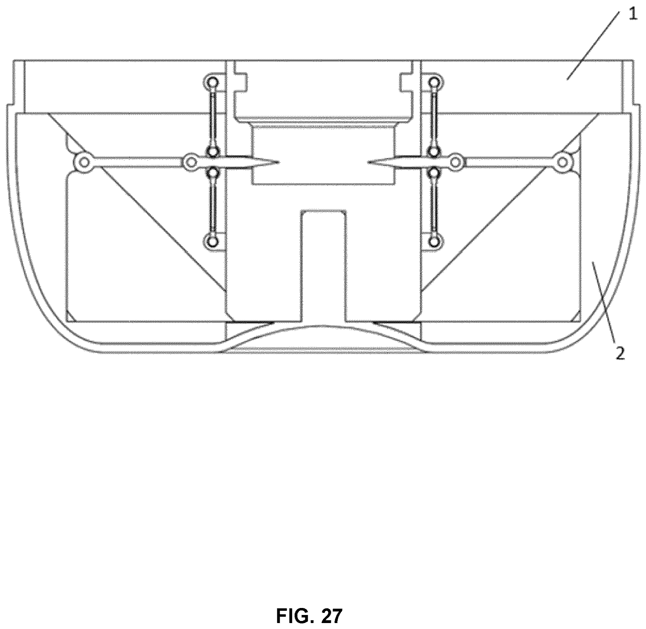

On the basis of Embodiment 1, another implementation method under a different conceptual approach is as follows: the drive structure is a linkage structure hinged with the piercing base 72. The linkage structure includes at least one main connecting rod 91 obliquely connected to the piercing base 72. One end of the main connecting rod 91 is hinged to the sidewall of the lower structure 2, and the other end of the main connecting rod 91 is hinged to the piercing base 72. When the upper structure 1 and the lower structure 2 undergo the longitudinal linear relative movement, the main connecting rod 91 drives the piercing needles 3 to pierce a capsule in the capsule chamber 111 along the piercing channel 12, referring to Figs. 22, 23, 26, and 27.

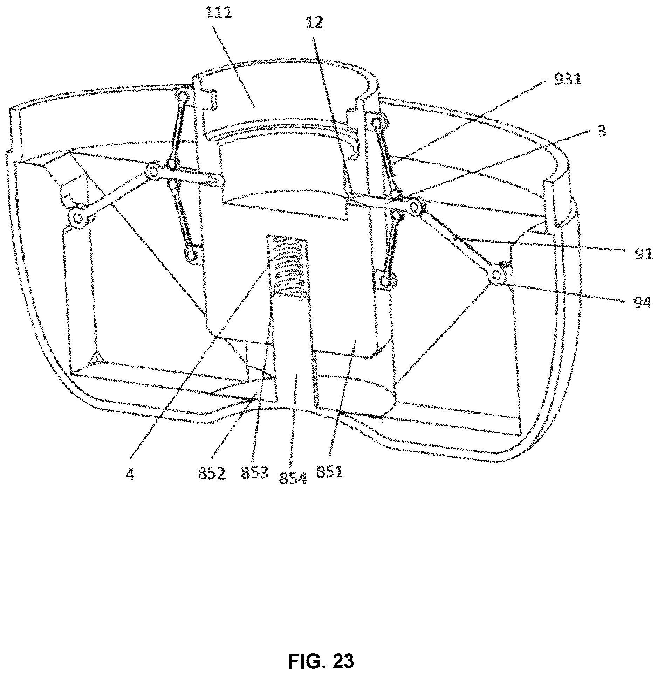

The main connecting rod 91 is tilted in a direction gradually tilting upwardly from the position at the upper edge of the lower structure 2 and toward the capsule chamber 111. This way can realize the change of the horizontal distance of the main connecting rod 91 during the upward movement of the lower structure 2 and realize the piercing movement of the piercing base 72 toward the capsule chamber 111 when the lower structure 2 moves relatively upward, see Fig. 23.

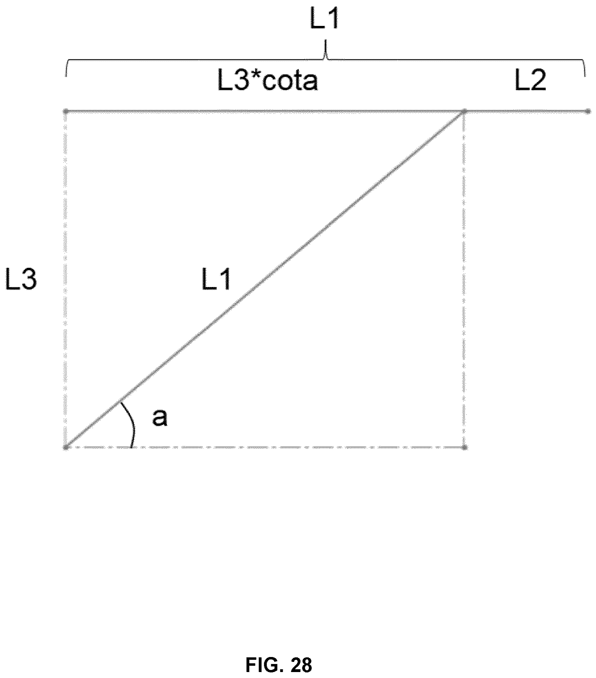

In some embodiments, the direction of the piercing channel 12 is the horizontal direction or an inclined upward or inclined downward direction. Taking the direction of the piercing channel 12 being a horizontal direction as an example, a relationship between the incline angle a of the main connecting rod 91, the length L1 of the main connecting rod 91, the piercing distance L2, and the longitudinal linear movement distance L3 of upper structure 1 and the lower structure 2 is represented as L1≥L2+L3*cota, referring to Fig. 28. This setting can determine the remaining indicators after determining any three indicators according to the need. In some embodiments, the piercing base 72 includes the piercing needles 3 provided with a connecting portion of connecting rod 94 or a needle holder 31 with the piercing needles 3. Preferably, the piercing base 72 is piercing needles 3 provided with the connecting portion of connecting rod 94. The piercing needles 3 are contained in a portion of the piercing channel 12 at any time, which ensures that the piercing needles 3 move along the piercing channel 12, referring to Fig. 23.

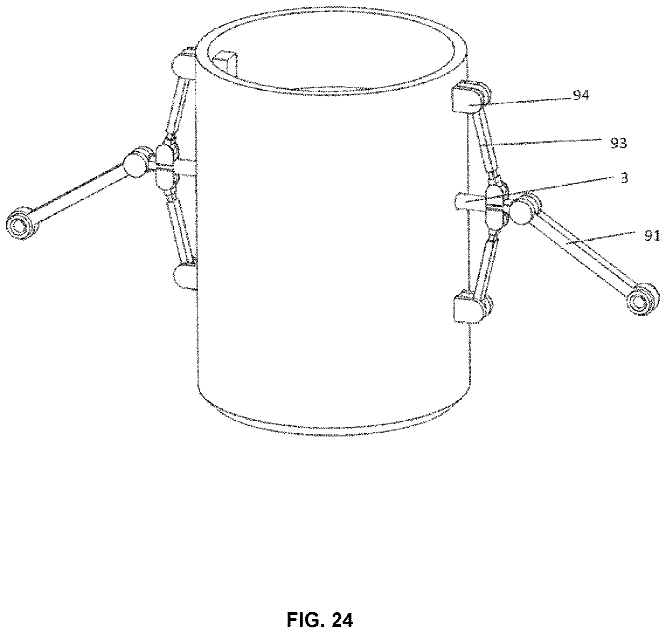

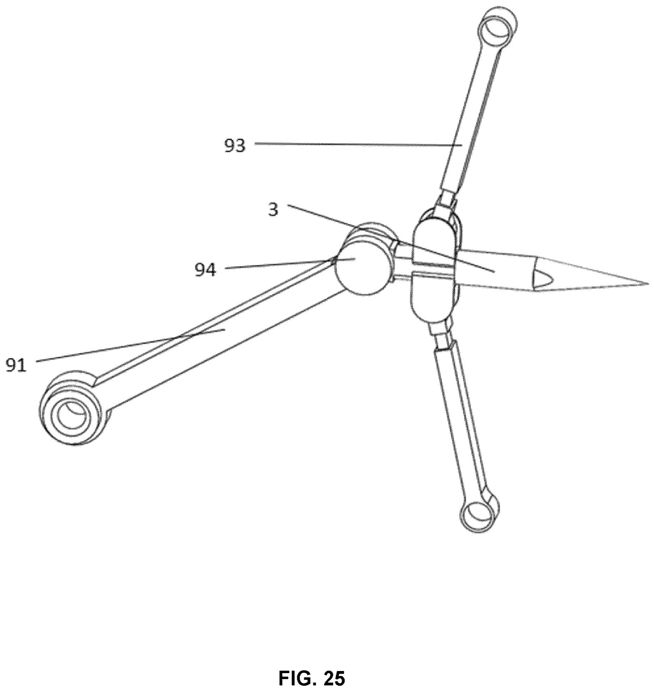

The inhaler further includes a direction maintaining structure for maintaining that the main connecting rod 91 moves in a plane. One embodiment of the direction maintenance structure is as follows: the direction maintaining structure is a running channel 921 that has the same thickness or diameter as the main connecting rod 91, the running channel 921 being formed by two plate structures 922 extending out on the side of the lower structure 2. The direction maintaining structure may also be two maintaining connecting rods 93 connected to the upper structure 1, the two maintaining connecting rods 93 are provided on both sides of the piercing channel 12, one end of each of the maintaining connecting rods 93 is pivotally connected (e.g., hinged) to the sidewall of the upper structure 1, the other end of each of the maintaining connecting rods 93 is movably connected to the piercing needles 3 or the needle holder 31, and the two maintaining connecting rods 93 are symmetrically provided around the piercing channel 12. The arrangement of the maintaining connecting rod 93 and the main connecting rod 91 also serves as an anti-separation structure, see Figs. 22, 24, and 25.

### Embodiment 6

On the basis of Embodiments 1-5, the following embodiments are added:

The bottom of the upper structure 1 and the bottom surface of the lower structure 2 are provided with mutually socketed lumen structures in correspondence, and the lumen structures include an upper lumen 41 at the bottom of the upper structure 1 and a lower lumen 42 extending from the bottom surface of the lower structure 2, the upper lumen 41 is socketed on the outer side of the lower lumen 42 or the upper lumen 41 extends into the inner side of the lower lumen 42, and the reset spring 4 is provided in the center of the upper lumen 41 and the lower lumen 42, referring to Figs. 2 and 8.

The inhaler further includes a direction-guiding structure for maintaining a longitudinal linear movement between the upper structure 1 and the lower structure 2, the direction-guiding structure includes a direction-guiding groove or a direction-guiding column 572 arranged in the middle of the upper structure 1, and the direction-guiding structure further includes a direction-guiding column or a direction-guiding groove 571 arranged in the middle of the lower structure 2 in correspondence, the direction-guiding groove and the direction-guiding column arranged in different structures are adapted to each other, and the direction of the direction-guiding groove and the direction-guiding column is a longitudinal straight line. A better embodiment is as follows: the lower lumen 42 is socketed inside the upper lumen 41, and the direction-guiding column is provided on the outside of the outer sidewall of the upper lumen 41, referring to Figs. 2, 5, and 9.

Another embodiment of the reset spring 4 and the guarantee of the longitudinal linear movement is as follows: a bottom cavity 852 is provided within the lower structure 2 for the movement of the bottom structure 851 of the upper structure 1, and the bottom structure 851 is adapted to the bottom cavity 852 in shape, the reset spring 4 is provided within the bottom cavity 852, and a portion of the bottom structure 851 is always provided within the bottom cavity 852, which can also well realize guidance of the longitudinal linear movement. Preferably, the bottom structure 851 may be provided with a secondary adaptation cavity 853, a secondary adaptation rod 854 extends from the bottom cavity 852, and the secondary adaptation cavity 853 adapts to the secondary adaptation rod 854. The secondary adaptation rod 854 extends into the secondary adaptation cavity 853, and the reset spring 4 is provided in the secondary adaptation cavity 853. This arrangement can further strengthen the guidance of the movement direction and adaptation effect, ensuring the stability of movement, referring to Figs. 2 and 15.

### Embodiment 7

On the basis of Embodiments 1-6, the inhaler may also include an anti-separation structure, which can both ensure longitudinal linear movement and prevent separation of the upper structure 1 from the lower structure 2 after the upper structure 1 is combined with the lower structure 2. The anti-separation structure may be implemented in any locking manner. The anti-separation structure includes a limit rod 51 extending from below the upper structure 1, a locking protrusion 52 is provided at the lowermost part of the limit rod 51, the length of the limit rod 51 is not less than the longitudinal linear movement distance between the upper structure 1 and the lower structure 2, and the lower structure 2 is provided with a limit protrusion 54 adapted to the locking protrusion 52, and a position of the locking protrusion 52 contacting the limit protrusion 54 is an anti-separation position, and other positions are positions for maintaining the movement state. Preferably, a length of the limit rod 51 from the locking protrusion 52 to the lowermost end of the upper structure 1 is equal to the longitudinal linear movement distance between the upper structure 1 and the lower structure 2. This setting prevents shaking. A movement groove 53 for movement of the locking protrusion 52 is correspondingly provided on the sidewall of the lower structure 2, and a limit protrusion 54 adapted to the locking protrusion 52 is provided on the uppermost part of the movement groove 53, and the length of the movement groove 53 is equal to the length of the limit rod 51. Preferably, the limit protrusion 54 is a protruding structure protruding from the sidewall of the lower structure 2, or the limit protrusion 54 is a top body from the bottom of the movement groove to the sidewall of the lower structure 2 provided at the top of the movement groove 53, as shown in Figs. 5-7.

The more preferred embodiment is as follows: a guide groove 55 is provided above the movement groove 53, a direction and a width of the guide groove 55 are identical to a direction and a width of the movement groove 53, a lowermost end of the guide groove 55 is an uppermost part of the limit protrusion 54, a guide surface 56 is provided at the uppermost part of the limit protrusion 52, which helps the limit rod 51 to enter the guide groove 55 more easily. To ensure stability and balance of the setting, at least one pair of limit rods 51 and corresponding movement grooves 53 are provided symmetrically on the left and right sidewalls or on front and rear sidewalls to ensure stable and balanced arrangement. Preferably, two pairs of limit rods 51 and corresponding movement grooves 53 may be symmetrically provided around the capsule chamber 111 on the left and right sidewalls or on the front and rear sidewalls, referring to Figs. 5-7.

Another embodiment of the anti-separation structure is as follows: the anti-separation structure includes a connecting structure that interconnects without interfering with the longitudinal linear movement. Preferably, the connecting structure is an interconnecting link, and the interconnecting link is provided with connecting portions at the connection with both the upper structure 1 and the lower structure 2. For example, the interconnecting link may be four interconnected diamond-shaped links, both side ends of which are free, and the upper and lower sides of which are respectively connected with the upper structure 1 and the lower structure 2. As another example, the interconnecting link includes at least one main connecting rod 91 connected to the side edge of the lower structure 2, the other end of the main connecting rod 91 is connected to the piercing structure, and the piercing structure always includes a portion of the piercing needles within a piercing channel. In this way, it is possible to realize not only the anti-separation, but also the piercing movement of the piercing needles by the longitudinal linear relative movement through the main connecting rod 91. The main connecting rod 91 moves in a plane, referring to Fig. 15.

### Embodiment 8

On the basis of Embodiments 1-7, the inhaler may also be provided with a structure that facilitates operation, the specific embodiment is as follows: in a stationary state, an anastomosis table 612, shaped in accordance with the upper cavity of the lower structure 2, is provided above the upper structure 1, an upper plane of the anastomosis table 612 is parallel to an upper plane of the upper cavity of the lower structure 2, the anastomosis table 612 is able to move perpendicularly in and out of the upper cavity, and an operation table 61 is provided above the anastomosis table 612, and finger placement structures are provided on both sides of the operation table 61.

A positional relationship between the anastomosis table 612 and the operation table 61 is set as the distance between the anastomosis table 612 and the operation table 61 being not less than the longitudinal linear movement distance between the upper structure 1 and the lower structure 2, which ensures an easy operation and prevents the operation table 61 from entering the upper cavity of the lower structure 2, which is not good for operation. In some embodiments, the operation table 61 has the same peripheral shape as the anastomosis table 612.

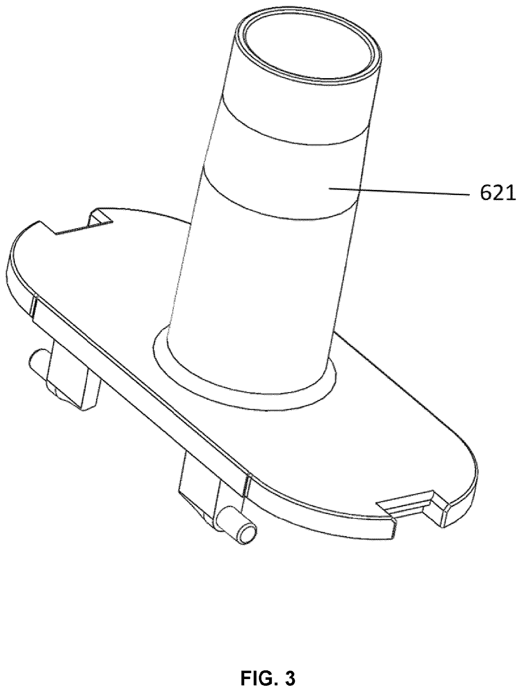

In some embodiments, an inhalation channel section 62 with a drug inhalation channel is also provided on the upper structure 1, a nozzle section 63 with an anastomosis suction nozzle is also sleeved on the inhalation channel section 62, the bottom of the inhalation channel section 62 and the bottom of the nozzle section 63 are provided with peripheral arc structures consistent with the shape of the anastomosis table 612, and the bottom of the uppermost nozzle section 63 is the operation table 61. This arrangement ensures that the combined structure is neat and stable and the hand placement operation is accomplished through the arc structure on the nozzle section 63. An anti-slip pattern 611 is provided on the arc structure of the operation table 61 to increase the friction and controllability during operation, referring to Figs. 2, 3, and 8.

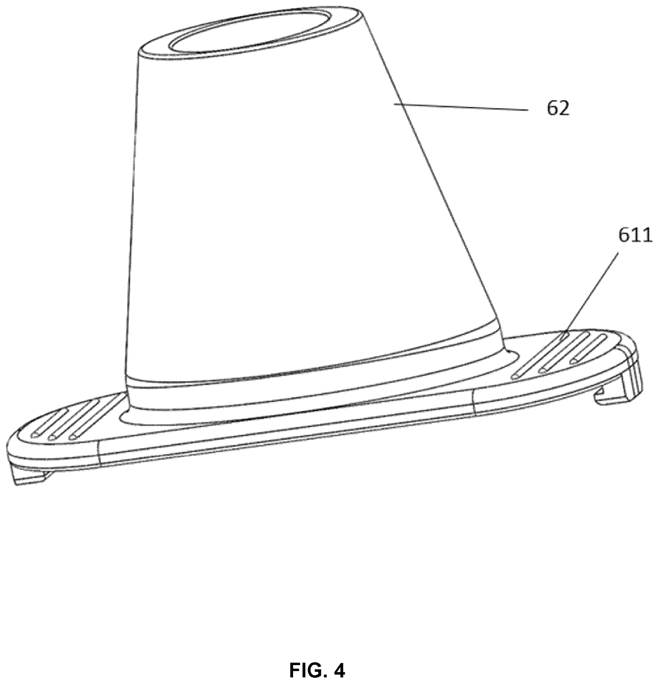

Further, to ensure sufficient disclosure, the combination of the inhalation channel section 62 with the other embodiments of the present disclosure is described as follow. The inhalation channel section 62 with the drug inhalation channel is also provided on the upper structure 1, and the inhalation channel is provided on the upper structure 1 through a rotational combination or a snap-on combination. The inhalation channel section 62 is provided with a columnar channel tube 621. A barrier mesh is provided at the bottom of the inhalation channel section 62, and the barrier mesh is configured to prevent the capsule from being inhaled during inhalation of the dry powder. The nozzle section 63 with an anastomosis suction nozzle is also sleeved over the inhalation channel section 62, and an anastomosis tube 631 that is consistent with the columnar channel tube 621 is internally arranged above the nozzle section 63. This setting increases the adaptation degree between the nozzle section 63 and the inhalation channel section 62. The bottom of the inhalation channel section 62 and the bottom of the nozzle section 63 are provided with an arc structure that is consistent with a shape of the operation table 61, which ensures that the combined structure is neat and stable. The hand placement operation is accomplished through the arc structure on the nozzle section 63, and the anti-slip pattern 611 is provided on the arc structure on the nozzle section 63 to increase the friction and controllability in the operation process. A vertically snapping protective cap 64 or a rotationally opening and closing protective cap 64 is also provided above the nozzle section 63 to prevent contamination, referring to Figs. 1-4. By placing the index finger and the middle finger at the finger placement structure and placing the thumb at a bottom of the lower structure 2, it can complete the relative movement between the upper structure 1 and the lower structure 2 through cooperation of the three fingers, thereby realizing the purpose of piercing the capsule from the side by the longitudinal linear movement.

The description of the above embodiments is only for understanding the present disclosure. It should be noted that, for a person of ordinary skill in the art, there are a number of improvements that can be made to the present disclosure without departing from the principles of the present disclosure, which will also fall within the protection of the claims of the present disclosure.

## Claims

1. A dry powder inhaler, comprising:
a piercing base, the piercing base being provided with one or more piercing needles for piercing a capsule from a side;
an upper structure, the upper structure being formed with a capsule chamber and a piercing channel, wherein
the capsule chamber is configured for accommodating the capsule;
the piercing channel is arranged laterally outside of the capsule chamber and connected to the capsule chamber, the piercing needles undergo a lateral piercing movement along the piercing channel;
a lower structure for accommodating a portion of the upper structure, the upper structure being subjected to a longitudinal linear movement relative to the lower structure; and
a drive structure arranged on at least one of the upper structure and the lower structure, the piercing base being driven by the drive structure to undergo movement; wherein
the drive structure moves with the upper structure and the lower structure undergoing a longitudinal linear relative approach movement, the drive structure drives the piercing base to move in a direction of the piercing channel, the piercing needles move with the piercing base along the piercing channel to pierce the capsule in the capsule chamber; the drive structure moves with the upper structure and the lower structure undergoing a longitudinal linear relative separation movement, the drive structure drives the piercing base to move in the direction of the piercing channel, and the piercing needles leave the capsule chamber with the piercing base along the piercing channel.

2. The dry powder inhaler according to claim 1, wherein the piercing channel is a straight channel and the piercing needles are straight needles; and the piercing needles are contained in a portion of the piercing channel at any time.

3. The dry powder inhaler according to claim 2, wherein the drive structure includes
an inclined track arranged within the lower structure for a tilting movement of the piercing base, the inclined track is tilted in a direction gradually inclined from a position at an upper edge of the lower structure to a lower portion of the lower structure and toward the capsule chamber, when the upper structure and the lower structure are subjected to the longitudinal linear relative approach movement, the piercing base is driven by the inclined track and guided by the piercing channel to move toward the capsule chamber, and the piercing needles enter the capsule chamber along the piercing channel to complete piercing of the capsule.

4. The dry powder inhaler according to claim 3, wherein
the direction of the piercing channel is horizontal, a length of the inclined track is L1, a piercing distance of the piercing needles is L2, and a longitudinal linear movement distance between the upper structure and the lower structure is L3, and a relationship between the length L1, the piercing distance L2, and the longitudinal linear movement distance L3 is represented as that the length L1 is greater than a length of a hypotenuse of a right triangle formed by L2 and L3.

5. The dry powder inhaler according to claim 4, wherein the inclined track is groove-type inclined tracks provided on front and rear sidewalls of the lower structure, the piercing base is provided with protruding columns extending into the groove-type inclined tracks, and one piercing base is arranged around the capsule chamber;
the inhaler is of single-side piercing type, two groove-type inclined tracks are provided on the front and rear sidewalls of the lower structure, the protruding columns extending into the two groove-type inclined tracks are provided on front and rear sides of the piercing base, the piercing base is provided with two piercing needles arranged in parallel, and two piercing channels arranged in parallel are provided on the same side in correspondence.

6. The dry powder inhaler according to claim 5, wherein the inclined track is groove-type inclined tracks provided on front and rear sidewalls of the lower structure, the piercing base is provided with protruding columns extending into the groove-type inclined tracks, and two piercing bases are arranged around the capsule chamber;
the inhaler is of double-side piercing type, four groove-type inclined tracks are provided correspondingly on the front and rear sidewalls of the lower structure, and the protruding columns extending into the two groove-type inclined tracks are provided on front and rear sides of the piercing base.

7. The dry powder inhaler according to claim 5, wherein the two piercing bases and the four groove-type inclined tracks are symmetrically provided around the capsule chamber, one piercing needle is provided on one piercing base, and two piercing channels arranged in parallel or in the same straight line are provided on both sides of the inhaler.

8. The dry powder inhaler according to claim 5, further comprising
a motion-guiding structure for ensuring movement of the piercing base along the piercing channel, the piercing base is provided with an adapting portion in combination with the motion-guiding structure, and the adapting portion moves on the motion-guiding structure;
wherein the motion-guiding structure is a cylindrical body or a columnar cavity extending from left and right sidewalls of the upper structure or the lower structure, and a direction of the motion-guiding structure is parallel to the piercing channel.

9. The dry powder inhaler according to claim 8, wherein the motion-guiding structure is the cylindrical body or the columnar cavity extending from a sidewall of the upper structure;
when the motion-guiding structure is the cylindrical body, the adapting portion is a columnar cavity sleeved on an outside of the cylindrical body, the protruding columns extend from an outside of the front and rear walls of the columnar cavity, or
when the motion-guiding structure is the columnar cavity, the adapting portion is a cylindrical body arranged inside the columnar cavity, the protruding columns extend from an outside of the front and rear walls of the cylindrical body, and transverse penetration notches are provided on the front and rear sidewalls of the columnar cavity to realize extending of the protruding columns and ensure protruding movement.

10. The dry powder inhaler according to claim 9, wherein secondary adaptation grooves are provided in the columnar cavity and secondary adaptation protrusions are provided on the cylindrical body, and the secondary adaptation grooves are provided symmetrically on an inside of the front and rear sidewalls or an inside of upper and lower sidewalls of the columnar cavity.

11. The dry powder inhaler according to claim 5, wherein a guide opening is provided at the uppermost portion of the lower structure, the guide opening is larger than an opening of the inclined track, and the guide opening is a triangular opening.

12. The dry powder inhaler according to claim 3, wherein the inclined track is a slope-type inclined track provided on left and right sidewalls of the lower structure, a movement track of the slope-type inclined track is an inclined surface of the slope-type inclined track, and the piercing base is provided with an anastomosing surface that fits into the inclined surface of the slope-type inclined track.

13. The dry powder inhaler according to claim 12, wherein when the inhaler is of single-side piercing type, the inhaler includes a slope-type inclined track and the piercing base, wherein two piercing needles arranged in parallel are provided on the piercing base, and two piercing channels arranged in parallel are provided on the same side of the dry powder inhaler; or
when the inhaler is of double-side piercing type, two slope-type inclined tracks are symmetrically provided on the left and right sidewalls of the lower structure and two piercing bases corresponding to the two slope-type inclined tracks are provided, one piercing needle is provided on the piercing base, and two piercing channels arranged in parallel or in the same straight line are provided on both sides of the dry powder inhaler.

14. The dry powder inhaler according to claim 13, further comprising:
a fitting structure for fitting a main base on the inclined track, wherein
the fitting structure includes a spring structure providing elasticity on side, one end of the spring structure being attached to a lower end of the upper structure and the other end of the spring structure being attached to a side of the main base; or
the fitting structure is a T-shaped limiting groove arranged on the inclined surface, and the fitting structure further includes an adapted T-shaped protrusion extending from the anastomosing surface.

15. The dry powder inhaler according to claim 2, wherein
the drive structure includes a linkage structure connected to the piercing base; and a hinge structure configured to articulate the linkage structure with the lower structure and the piercing base; wherein
the linkage structure includes at least one main connecting rod obliquely connected to the piercing base, one end of the main connecting rod is hinged to a sidewall of the lower structure through the hinge structure, and the other end of the main connecting rod is hinged to the piercing base through the hinge structure;
the main connecting rod is tilted in a direction gradually tilting upwardly from a position at the upper edge of the lower structure and toward the capsule chamber; and
when the upper structure and the lower structure undergo the longitudinal linear relative movement, the main connecting rod rotates with the longitudinal linear relative movement, the main connecting rod rotates to drive the piercing base to move in the direction of the piercing channel, and the piercing needles enter the capsule chamber along the piercing channel with the piercing base to pierce the capsule in the capsule chamber.

16. The dry powder inhaler according to claim 15, wherein
the direction of the piercing channel is horizontal, an incline angle of the main connecting rod is a, a length of the main connecting rod is L1, a piercing distance of the piercing needles is L2, and a relationship between the incline angle a of the main connecting rod, the length L1 of the main connecting rod, the piercing distance L2, and the longitudinal linear movement distance L3 of the upper structure and the lower structure is represented as L1≥L2+L3/cota.

17. The dry powder inhaler according to claim 16, further comprising a direction maintaining structure for maintaining that the main connecting rod moves in a plane, wherein
the direction maintaining structure is a running channel that has the same thickness or diameter as the main connecting rod, and the running channel is formed by two plate structures extending out on the side of the lower structure; or
the direction maintaining structure is a structure of two maintaining connecting rods connected to the upper structure, the two maintaining connecting rods are provided on both sides of the piercing channel, one end of each of the maintaining connecting rods is hinged to the sidewall of the upper structure, the other end of each of the maintaining connecting rods is movably connected to the piercing needles or the needle holder, and the two maintaining connecting rods are symmetrically provided around the piercing channel.

18. The dry powder inhaler according to claim 1, wherein the lower structure is provided with an accommodating chamber, and the upper structure undergoes the longitudinal linear relative movement within the accommodating chamber of the lower structure,
in a stationary state, an anastomosis table shaped in line with an upper cavity of the lower structure is provided above the upper structure, an upper plane of the anastomosis table is horizontal to an upper plane of the upper cavity of the lower structure, the anastomosis table is perpendicularly movable in and out of the upper cavity, an operation table is provided above the anastomosis table, and finger placement structures are provided on both sides of the operation table.

19. The dry powder inhaler according to claim 18, wherein a distance between the anastomosis table and the operation table is not less than a longitudinal linear movement distance between the upper structure and the lower structure.

20. The dry powder inhaler according to claim 1, further comprising an anti-separation structure, wherein the anti-separation structure both ensures the longitudinal linear movement and prevents separation of the upper structure from the lower structure after the upper structure and the lower structure are combined; the anti-separation structure includes a limit rod extending from below the upper structure, a locking protrusion is provided at the lowermost part of the limit rod, a length of the limit rod is not less than a longitudinal linear movement distance between the upper structure and the lower structure, the lower structure is provided with a limit protrusion adapted to the locking protrusion, a position of the locking protrusion contacting the limit protrusion is an anti-separation position, and other positions except for the anti-separation position are positions for maintaining movement state.

21. The dry powder inhaler according to claim 20, wherein a length of the limit rod from the locking protrusion to the lowermost end of the upper structure is equal to the longitudinal linear movement distance between the upper structure and the lower structure.

22. The dry powder inhaler according to claim 20, wherein the limit protrusion is a protruding structure extending from a sidewall of the lower structure.

23. The dry powder inhaler according to claim 20, wherein the lower structure is provided with a movement groove for movement of the locking protrusion, the limit protrusion is a top body from a bottom of the movement groove to a sidewall of the lower structure, and the top body is provided at a top of the movement groove.

24. The dry powder inhaler according to claim 23, wherein a guide groove is provided above the movement groove, a direction and a width of the guide groove are identical to a direction and a width of the movement groove, a lowermost end of the guide groove is an uppermost part of the limit protrusion, and a guide surface is provided at the uppermost part of the limit protrusion.

25. The dry powder inhaler according to claim 20, further comprising an anti-separation structure, wherein the anti-separation structure both ensures the longitudinal linear movement and prevents separation of the upper structure from the lower structure after the upper structure and the lower structure are combined; the anti-separation structure includes a connecting structure interconnecting without interfering with the longitudinal linear movement, the connecting structure is an interconnecting link, and the interconnecting link is provided with connecting portions at the connection with both the upper structure and the lower structure.

26. The dry powder inhaler according to claim 25, wherein the interconnecting link is a structure of four interconnected diamond-shaped links, both side ends of which are free, and the upper and lower sides of which are respectively connected with the upper structure and the lower structure.

27. The dry powder inhaler according to claim 26, wherein the interconnecting link includes at least one main connecting rod connected to a side edge of the lower structure, the other end of the main connecting rod is connected to a piercing structure, and the piercing structure always includes a portion of the piercing needles within the piercing channel.

28. The dry powder inhaler according to claim 20, wherein a reset structure is provided at a bottom of the upper structure and the lower structure to make the locking protrusion contact with the limit protrusion in an unused state, wherein the reset structure is a reset spring.

29. The dry powder inhaler according to claim 28, wherein mutually socketed lumen structures are provided at the bottom of the upper structure and at a bottom surface of the lower structure in correspondence, the lumen structures are an upper lumen at the bottom of the upper structure and a lower lumen extending from the bottom surface of the upper structure, the upper lumen is socketed on an outer side of the lower lumen or the upper lumen extends into an inner side of the lower lumen, and the reset spring is provided in a center of the upper lumen and the lower lumen; or
a bottom cavity for movement of a bottom structure of the upper structure is provided within the lower structure, the bottom structure is adapted to the bottom cavity in shape, the reset spring is provided in the bottom cavity, and a portion of the bottom structure is provided within the bottom cavity.

30. The dry powder inhaler according to claim 1, further comprising: a direction-guiding structure for maintaining the longitudinal linear movement between the upper structure and the lower structure, wherein the direction-guiding structure includes a direction-guiding groove or a direction-guiding column provided in a middle of the upper structure, the direction-guiding structure further includes a direction-guiding column or a direction-guiding groove provided in a middle of the lower structure in correspondence, the direction-guiding groove is adapted to the direction-guiding column, and directions of the direction-guiding groove and the direction-guiding column are in a longitudinal straight line.
